# EUROPEAN PATENT SPECIFICATION

(11) **EP 2 359 742 B1**
(45) Date of publication and mention of the grant of the patent: **17.07.2013**
(21) Application number: 10831367.7
(22) Date of filing: 26.07.2010
(51) Int. Cl.: A61B 1/00, A61B 1/04, A61B 5/07

(54) **MEDICAL DEVICE SYSTEM, CAPSULE MEDICAL DEVICE SYSTEM, AND METHOD FOR DISPLAYING POSTURE ITEMS OF BODY TO BE TESTED**
MEDIZINGERÄTESYSTEM, EINGEKAPSELTES MEDIZINGERÄTESYSTEM UND VERFAHREN ZUR ANZEIGE VON POSITIONIERUNGSSTELLEN AM ZU TESTENDEN KÖRPER
SYSTÈME DE DISPOSITIF MÉDICAL, SYSTÈME DE DISPOSITIF MÉDICAL À CAPSULE, ET PROCÉDÉ POUR AFFICHER DES ÉLÉMENTS DE POSTURE D'UN CORPS À TESTER

(30) Priority: 19.11.2009 JP 2009264276
(43) Date of publication of application: 24.08.2011
(73) Proprietor: OLYMPUS MEDICAL SYSTEMS CORP., Tokyo 151-0072 (JP)
(72) Inventor: CHIBA, Atsushi, Tokyo 151-0072 (JP); KATAYAMA, Miho, Tokyo 151-0072 (JP); NISHIYAMA, Takeshi, Tokyo 151-0072 (JP)
(74) Representative: Gunzelmann, Rainer
(86) International application number: PCT/JP2010/062553
(87) International publication number: WO 2011/061968

(56) References cited:
- EP-A1- 2 110 067
- EP-A1- 2 143 371
- WO-A1-2008/096744
- WO-A1-2010/005818
- JP-A- 2006 288 832
- JP-A- 2007 195 961
- JP-A- 2009 213 613
- US-A- 5 211 167
- US-A1- 2002 091 331
- US-A1- 2009 054 796

## Description

### Field

The present invention relates to a medical apparatus system that acquires information on a subject, a capsule medical apparatus system, and a method of displaying a posture item of a subject.

### Background

In recent years, systems have been proposed in which in-vivo images are taken using a capsule endoscope, data of the taken in-vivo images is received by a receiving device, and the data of the in-vivo images received by the receiving device is displayed on an image display device (see Patent Literature 1). In the capsule endoscope system, a doctor observes the in-vivo images that are displayed on the image display device and performs an in-vivo diagnosis on the subject.

### Citation List

### Patent Literature

Patent Literature 1: International Publication No. WO 07/077922

US 2002/0091331 A1 relates to an electrocardiograph system eliminating the effects of posture changes for proper diagnosis. To compare cardiograms of a subject with reference cardiograms, a cardiogram storage device stores cardiograms and posture information, i.e., a piece of information (pointer) for specifying a portion that corresponds to the reference cardiogram from series of cardiograms stored. In a monitoring device, postures are displayed and can be selected to a display reference cardiograms as static images while displaying wave forms of the cardiograms received in real time as a time-varying.

US 2009/0054796 A1 relates to implantable medical devices, such as implantable cardiac stimulus device. To determine whether a particular patient is well suited to receive a particular implantable stimulus device, a pre-operative patient screening tool is provided including a stencil designed for comparison to a printed ECG. On a display screen a trace is shown and optionally a comparison shape is also shown. The use of such a display screen allows a practitioner to show to a patient how the trace compares to the shape which is chosen from a menu in order to match amplitude to a captured event.

US 5,211,167 relates to an ultrasonic diagnosing apparatus for displaying an ultrasonic image. An operator or doctor manually selects one of a plurality of stored posture marks. Image signal representing the marks are supplied to a mixing circuit and are mixed with the ultrasonic image signal to produce a composite image signal of the ultrasonic image signal and the mark signal. The composite image signal may be transmitted from a testing room to a diagnosing room in which one or more doctors are existing and the composite image may be displayed on a monitor provided in the diagnosing room or the composite image signal may be recorded on a video tape. To select the posture mark, a switch is operated to display a group of posture marks. A desired posture mark is then selected by operating a particular mark selection switch resulting in the display of the mark read out of a mark memory.

EP 2 110 067 A1 relates to an in-vivo information acquiring system using a capsule endoscope. An examination-procedure display unit displays a posture image indicating a posture to be taken by the subject who has the capsule endoscope in his internal organ. A storage unit stores acquired in-vivo images and received field strength information of each of a plurality of receiving antennas in a storage unit.

Post-published EP 2 143 371 A1 relates to a system for magnetically guiding a capsule medical device introduced into an organ of a subject. A display unit displays various pieces of information useful in operation of the magnetic guidance of the capsule medical device, including a body posture setting unit which is a graphical user interface for setting the body posture of the subject on a bed. Clicking on a body posture setting menu of a input unit, a body posture can be input, so that an image processor generates a subject pattern image shown these specified body posture

Post-published WO 2010/005818 A1 relates to electrical simulation generators in an implantable medical device (IMD). The IMD can re-orient an activity sensor or posture state module by prompting the patient to select an appropriate posture state indication. The patient needs to assume different posture states and select the corresponding posture state indication as prompted by the user interface. Conceptual diagrams illustrating different posture state indications representing the patient posture state are displayed on a screen.

### Summary

### Technical Problem

Conventional capsule endoscope systems capture in-vivo images of a subject whose posture has been changed in accordance with a region to be diagnosed in the subject. Such conventional capsule endoscope systems, however, cannot confirm the posture for which an in-vivo image has been captured, thereby making it difficult to perform capturing operations for all postures necessary for diagnosis without omission. Further, since the conventional capsule endoscope systems do not store posture information of the subject in association with in-vivo image data, the doctor cannot confirm which posture the in-vivo image is captured for. In view of the foregoing, there is a need for a capsule endoscope system capable of reducing the work of the health care professional such as a doctor, a nurse, and a clinical laboratory technologist required for diagnosis.

The present invention has been made in view of the above problems, and an object of the invention is to provide a capsule endoscope system capable of reducing the work of the health care professional required for diagnosis.

### Solution to Problem

This object is solved by the present invention as claimed in the independent claims. Preferred embodiments ar defined in the dependent claims.

To solve the problems and achieve the object, a medical apparatus system according to an aspect of the present invention includes a medical apparatus that acquires information on a subject; a posture item display unit that displays a posture item of the subject in which the information on the subject is acquired; an input unit that inputs the posture item of the subject; and a display controller that, in response to the input of the input unit, changes a display mode of the posture item of the subject that is displayed by the posture item display unit.

To solve the problems and achieve the object, a capsule medical apparatus system according to an aspect of the present invention includes a capsule medical apparatus to be inserted into a subject to take an in-vivo image of the subject; an input unit that inputs information on a posture of the subject in which the in-vivo image is taken; a storage unit that stores the in-vivo image, which is taken by the capsule medical apparatus, in association with the information on the posture of the subject, which is input by the input unit; and an image display unit that displays the in-vivo image, which is stored in the storage unit, and the information on the posture of the subject, which is associated with the in-vivo image.

To solve the problems and achieve the object, a method of displaying a posture item of a subject according to an aspect of the present invention includes a display process of displaying a posture item of a subject in which a medical apparatus acquires information on the subject; and a display control process of changing, in response to an input of a posture item of the subject, a display mode of the posture item of the subject that is displayed on the posture item display unit.

### Advantageous Effects of Invention

A medical apparatus system and a method of displaying a posture item of a subject according to the present invention display a posture item of a subject in which the information on the subject is acquired and change a display mode of the subject in response to an input of a posture item of the subject, and thus can reduce the work of the health care professional required for diagnosis of the subject. Further, a capsule medical apparatus system displays posture information of the subject when in-vivo image is acquired, together with the in-vivo image, and thus can reduce the work of the health care professional required for diagnosis of the subject.

### Brief Description of Drawings

FIG. 1 is a schematic diagram of an entire configuration of a capsule endoscope system of an embodiment of the present invention.
FIG. 2 is a flowchart of a flow of a diagnostic process performed by a control device in FIG. 1.
FIG. 3 is a diagram of an example of a display screen of a posture item.
FIG. 4 is a diagram of an example of a display screen of a posture item.
FIG. 5 is a diagram of an example of a display screen of a posture item.
FIG. 6 is a diagram of an example of a capsule-image display screen.
FIG. 7 is a diagram of a modification of the capsule-image display screen in FIG. 6.
FIG. 8 is a diagram of a modification of the capsule-image display screen in FIG. 6.
FIG. 9 is a diagram of an example of a diagnostic display screen.

### Description of Embodiments

An entire configuration of a capsule endoscope system as an embodiment of the present invention will be described below with reference to the drawings.

### Entire configuration of capsule endoscope system

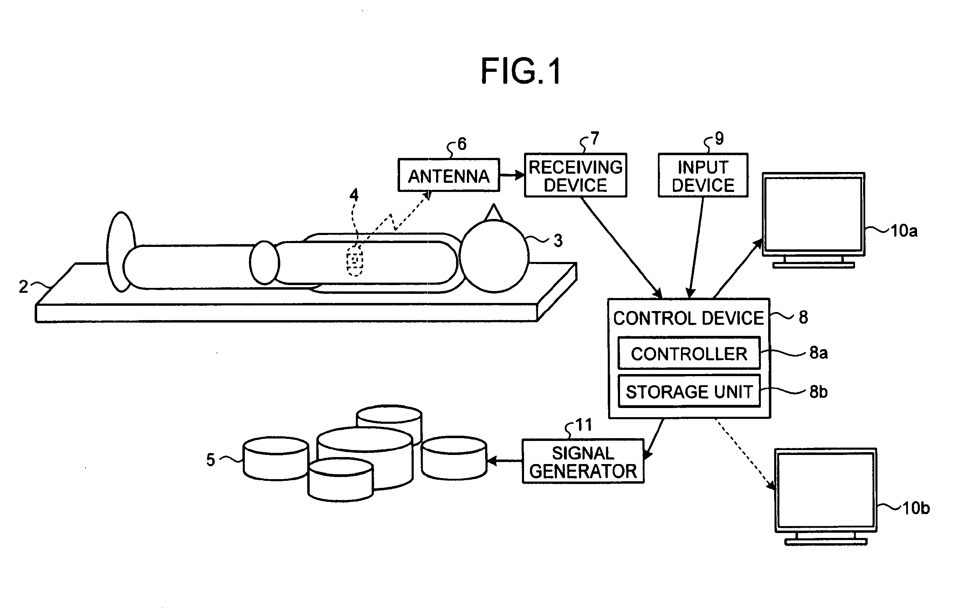

First, an entire configuration of the capsule endoscope system of the embodiment of the present invention will be described with reference to FIG. 1.

FIG. 1 is a schematic diagram of an entire configuration of the capsule endoscope system of the embodiment of the present invention. As illustrated in FIG. 1, a capsule endoscope system 1 of the embodiment of the present invention includes a bed 2; a capsule endoscope 4 serving as a capsule medical apparatus that is inserted together with a liquid, such as water or a normal saline solution, into a subject 3 laid on the bed 2 and then takes in-vivo images of the subject 3; a magnetic field generation device 5 that controls at least any one of the position and the posture of the capsule endoscope 4 that floats in the liquid; a receiving device 7 that receives data of the in-vivo images that are transmitted by radio from the capsule endoscope 4 via an antenna 6; and a control device 8.

The capsule endoscope 4 has an image capturing function of capturing an in-vivo image of a subject and a radio communication function for transmitting by radio various types of information, such as data of in-vivo images. The capsule endoscope 4 is formed to have a size such that it can be easily inserted into the subject 3, and the capsule endoscope 4 has a specific gravity approximately equal to or less than the specific gravity of the liquid, such as water or a normal saline solution. The capsule endoscope 4 sequentially takes in-vivo images at predetermined intervals of, for example, 0.5 second while moving in the subject 3 by peristalsis. The capsule endoscope 4 transmits the in-vivo images by radio. In the present embodiment, the capsule endoscope 4 includes imaging devices respectively at the ends of the capsule endoscope 4 in the longitudinal axis direction.

The magnetic field generation device 5 controls at least any one of the position and the posture of the capsule endoscope 4 in the subject 3. Specifically, the magnetic field generation device 5 generates a magnetic field to the capsule endoscope 4, which is inserted into the subject 3, and controls the movement of the capsule endoscope 4 in the liquid by using the magnetic force of the magnetic field. By controlling the movement of the capsule endoscope 4, the magnetic field generation device 5 controls at least any one of the position and the posture of the capsule endoscope 4 in the subject 3. In this case, the capsule endoscope 4 includes a magnet that causes a casing to move according to the magnetic field that is generated by the magnetic field generation device 5.

Operations of the magnetic field generation device are controlled by a signal generator 11 that operates according to control signals from the control device 8. Health care professionals control the movement of the capsule endoscope 4 on the basis of in-vivo images that are taken by one of the two imaging devices arranged in the capsule endoscope 4.

The antenna 6 is realized by using a loop antenna and is arranged on a predetermined position on the body surface of the subject 3. The number of antennas 6 to be arranged is not limited to one and multiple antennas may be arranged. The receiving device 7 receives the data of the in-vivo images, which is transmitted from the capsule endoscope 4 via the antenna 6, and outputs the received data of the in-vivo images to the control device 8. The control device 8 is realized by using a work station. The control device 8 includes a controller 8a and a storage unit 8b.

The controller 8a has a storage function of storing, for example, the data of the in-vivo images received by the receiving device 7, in the storage unit 8b; a display function of displaying, for example, the data of the in-vivo images received by the receiving device 7, on a capsule image display device 10a or a diagnostic display device 10b; a drive control function of outputting, to the signal generator 11, a control signal for controlling driving of the capsule endoscope 4; and an estimation function of estimating the position and the posture of the capsule endoscope 4 on the basis of the generated magnetic field that is estimated from the value of a signal output from the signal generator 11 and from the shape of the magnetic field generator 5.

The storage unit 8b stores the data of the in-vivo images, which are received by the receiving device 7, in association with information on the posture of the subject 3 during the image capturing operation and information on the position and the posture of the capsule endoscope 4 during the image capturing operation. The storage unit 8b also stores, with respect to each in-vivo region of the subject 3, information on posture items of the subject 3 in which observation should be performed and order information that defines the order of the posture items in which observations are performed.

An input device 9, such as a keyboard, a mouse pointer, and a joystick, is connected to the control device 8. By operating the input device 9, the health care professional inputs, to the control device 8, various types of operation input information for, for example, giving an instruction for moving the capsule endoscope 4 and acquiring captured images. The control device 8 functions as a display controller and an operation state confirmation unit according to the present invention. The storage unit 8b functions as a storage unit according to the present invention. The input device 9 functions as an input unit according to the present invention. The capsule image display device 10a functions as a posture item display unit according to the present invention. The diagnostic display device 10b functions as an image display unit according to the present invention.

### Diagnostic process

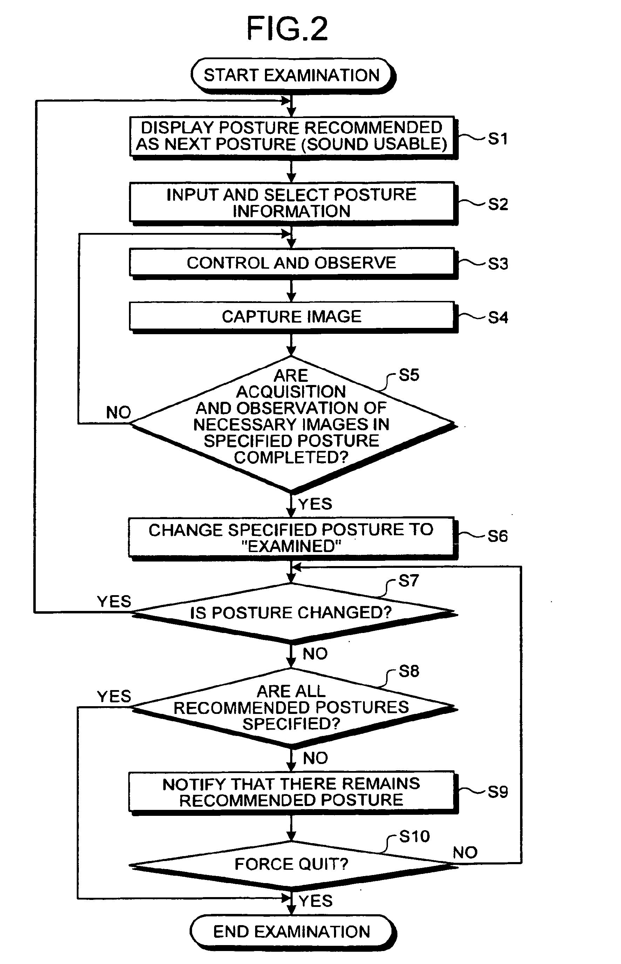

In the capsule endoscope system that has the above configuration, the control device 8 performs the following diagnostic process, which reduces the work of the health care professional required for diagnosis. A flow of the diagnostic process performed by the control device 8 will be described with reference to the flowchart of FIG. 2.

FIG. 2 is a flowchart of a flow of the diagnostic process performed by the control device 8. The flowchart in FIG. 2 starts at a timing when the health care professional gives an instruction for starting the diagnostic process to the control device 8 and the diagnostic process goes to the process at step S1.

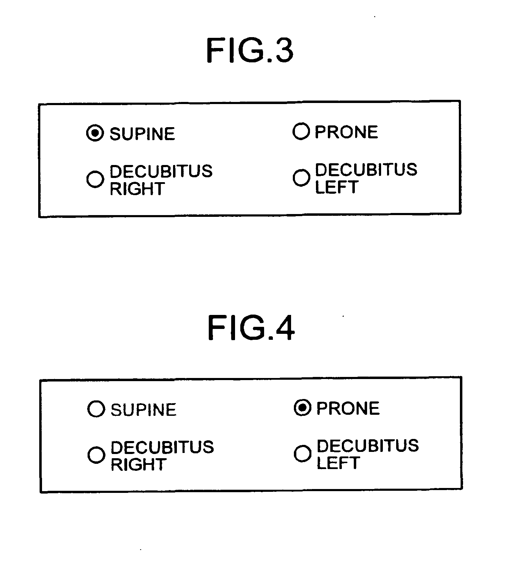

In the process at step S1, the control device 10 displays recommended posture items of the subject 3. When the process is after the posture of the subject 3 is changed, the control device 10 notifies a posture item that is not stored as "Examined" among the posture items that are displayed in the previous process. Specifically, the control device 8 notifies a posture item that is not stored as "Examined" by, on the basis of posture information that defines the order of posture items in which observations should be performed, changing the display color of the posture item in which observation should be performed next among the posture items in FIG. 4 or informing a posture in which observation should be performed next using a pop-up display or an audio output saying "Next is the Decubitus right". Accordingly, the process at step S1 is completed and the diagnostic process goes to the process at step S2.

In the process at step S2, the control device 8 operates the input device 9 to cause it to input the information on the posture of the subject 3 in which in-vivo images are to be observed. Specifically, the control device 8 displays, for example, as illustrated in FIG. 3, four posture items "Supine", "Prone", "Decubitus right", and "Decubitus left" on the capsule image display device 10a as the information on recommended postures of the subject 3. The control device 8 then operates the input device 9 to click a check box provided to each posture item, thereby inputting the information on the posture of the subject 3 in which observation is performed. The control device 8 promotes the diagnostic process to the process at step S3 in the timing when the input device 9 inputs the posture information on the subject 3.

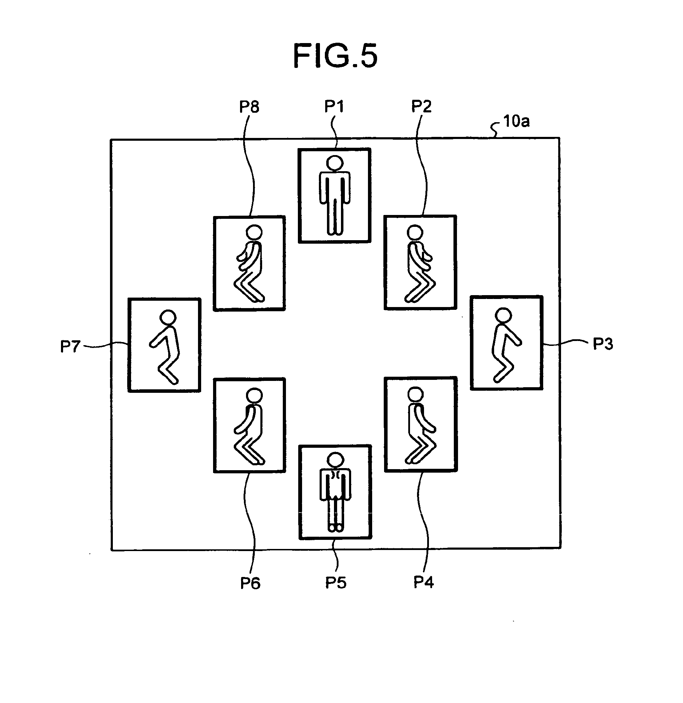

As illustrated in FIG. 5, the control device 8 may inform the recommended posture items of the subject 3 by displaying images P1 to P8 that schematically show the posture items of the subject 3. In FIG. 5, the image P1 is an image showing the posture item "Supine", the image P2 is an image showing a posture item between the posture item "Supine" and the posture item "Decubitus left", the image P3 is an image showing the posture item "Decubitus left", the image P4 is an image showing a posture item between the posture item "Decubitus left" and the "Prone", the image P5 is an image showing the posture item "Prone", the image P6 is an image showing a posture item between the posture item "Prone" and the posture item "Decubitus right", the image P7 is an image showing the posture item "Decubitus right", and the image P8 is an image showing a posture item between the posture item "Decubitus right" and the posture item "Supine".

In the image display example in FIG. 5, the control device 8 may, at the timing when the input device 9 inputs a posture item of the subject 3, change the display color of the image corresponding to the input posture item or display a name of the posture item near the image corresponding to the input posture item. Because of such a process, the input posture item can be easily recognized and a posture item can be input accurately. The control device 8 may, at the timing when the input device 9 selects an image, display the name of the posture item shown by the selected image.

When a posture item is selected, the control device 8 displays "Under examination" for the selected posture item. When a posture item is selected next, the control device 8 displays "Examined" for the already selected posture item and displays "Under examination" for the newly selected posture item.

In the process at step S3, in response to an operation of the input device 9, the control device 8 operates to guide the capsule endoscope 4 and to take in-vivo images and sequentially stores the taken in-vivo images in the storage unit 8b. Here, the control device 8 stores, in the storage unit 8b, the taken in-vivo images in association with information on the posture of the subject 3 during the image capturing operation and information on the position and the posture of the capsule endoscope 4 during the image capturing operation. Accordingly, the process at step S3 is completed and the diagnostic process goes to the process at step S4.

In the process at step S4, in response to an operation of the input device 9, the control device 8 captures the in-vivo images and stores the captured in-vivo images in the storage unit 8b as captured images. Here, the control device 8 stores, in the storage unit 8b, the captured images in association with the information on the posture of the subject 3 in which the captured images are taken and information on the position and the posture of the capsule endoscope 4 in which the captured images are taken. In the present embodiment, a posture item of the subject 3 in which observation is performed is input in the process at step S2. Alternatively, a posture item of the subject 3 may be input when an instruction for acquiring a captured image is given. Note that, in this case, only the information on the posture of the subject 3 in which the captured images are acquired is stored. Accordingly, the process at step 4 is completed and the diagnostic process goes to the process at step S5.

In the process at step S5, according to an operation of the input device 9, the control device 8 determines whether acquisition and observation of necessary in-vivo images are completed. When the result of the determination indicates that acquisition and observation of necessary in-vivo images are not completed, the control device 8 returns the diagnostic process to the process at step S3. In contrast, when acquisition and observation of necessary in-vivo images are completed, the control device 8 promotes the diagnostic process to the process at step S6. When the observation time in the same posture lasts for a predetermined time or more, the control device 8 may inform the information on the posture of the subject 3.

In the process at step S6, the control device 8 stores, as "Examined", the posture item of the subject 3 in which acquisition and observation of necessary in-vivo images are completed. Specifically, in the case where, for example, as illustrated in FIG. 3, the four posture items "Supine", "Prone", "Decubitus right", and "Decubitus left" are displayed as posture items of the subject 3 in which observation should be performed, when acquisition and observation of in-vivo images of the subject 3 whose posture is "Supine" are completed, the control device 8 changes the display mode of the posture item of the subject 3 that is stored as "Examined" by, for example, displaying "Supine" in a lighter display color as illustrated in FIG. 4. Because of such a process, the posture item in which acquisition and observation of necessary in-vivo images are completed can be visually confirmed. Accordingly, in all the posture items necessary for in-vivo regions to be observed, in-vivo images can be acquired and observed without omission. Accordingly, the process at step S6 is completed and the diagnostic process goes to the process at step S7.

In the process at step S7, according to an operation of the input device 9, the control device 8 determines whether the posture of the subject 3 is changed. A detection means, such as a weight sensor, may be arranged under the bed 2 to detect whether the posture of the subject 3 is changed. When the result of the determination indicates that the posture of the subject 3 is changed, the control device 8 returns the diagnostic process to the process at step S1. When the posture of the subject 3 is not changed, the control device 8 promotes the diagnostic process to the process at step S8. It is desirable that, when the posture of the subject 3 is changed, the control device 8 displays a display for confirming whether the information on the posture item, which is input in the process at step S2, is automatically reset or the posture item is reset. Such a process helps inputting a posture item again when the posture is changed and accordingly in-vivo images are taken according to an appropriate posture item.

In the process at step S8, the control device 8 determines whether all the posture items that are displayed in the process at step S1 are stored as "Examined". When the result of the determination indicates that all the posture items that are displayed in the process at step S1 are stored as "Examined", the control device 8 ends the diagnostic process. In contrast, when all the posture items that are displayed in the process at step S1 are not stored as "Examined", the control device 8 promotes the diagnostic process to the process at step S9.

In the process at step S9, the control device 8 notifies that there remains a posture item that is not stored as "Examined". Specifically, the control device 8 notifies that there remains a posture item that is not stored as "Examined" by changing the display color of the posture item that is not stored as "Examined" or informing that there is a recommended posture item by saying, for example, "An recommended posture item remains" using a pop-up display or an audio output. Accordingly, the process at step S9 is completed and the diagnostic process goes to step S10.

In the process at step S10, according to an operation of the input device 9, the control device 8 determines whether an instruction for ending the diagnostic process is given. When the result of determination indicates that an instruction for ending the diagnostic process is not given, the control device 8 returns the diagnostic process to the process at step S7. In contrast, when an instruction for ending the diagnostic process is given, the control device 8 ends the diagnostic process. If an instruction for ending the diagnostic process is given when acquisition and observation of in-vivo images are not completed in all the posture items that are displayed in the process at step S1, the control device 8 may give information that examinations in all the posture items are not completed.

### Configuration of capsule-image display screen

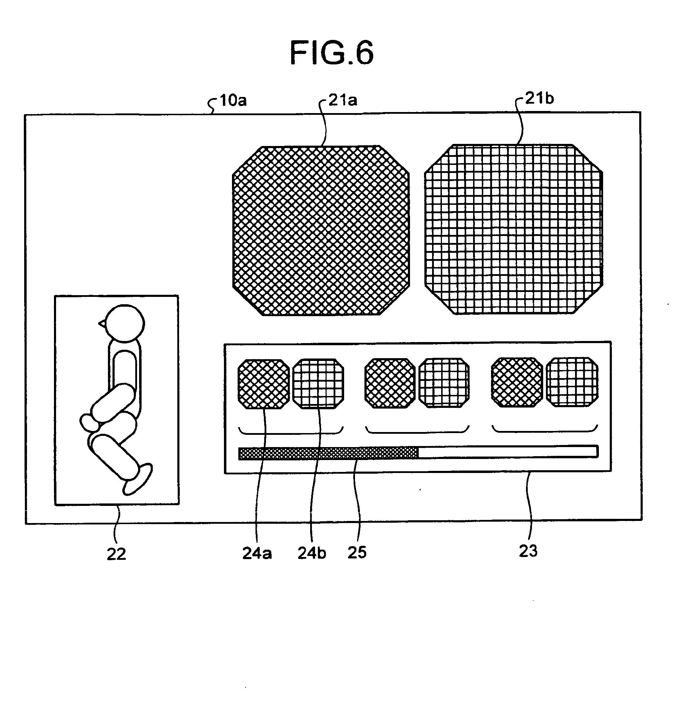

Next, a configuration of the capsule-image display screen that is displayed on the capsule image display device 10a in the above-described diagnostic process will be described with reference to FIG. 6.

FIG. 6 is a schematic diagram of a configuration of a capsule-image display screen that is displayed on the capsule image display device 10a. As illustrated in FIG. 6, the capsule-image display screen that is displayed on the capsule image display device 10a includes capsule image display areas 21a and 21b, a posture information display area 22, and a captured-image display area 23. The capsule image display areas 21a and 21b display in-vivo images that are taken by the two imaging devices of the capsule endoscope 4 in the process at step S2. The posture information display area 22 schematically displays a posture item that is associated with the in-vivo images displayed in the capsule image display areas 21a and 21b, i.e., the posture item of the subject 3 in which the in-vivo images that are displayed in the capsule image display areas 21a and 21b are taken. The captured-image display area 23 includes multiple captured images 24a and multiple captured images 24b that are captured by the two imaging devices of the capsule endoscope 4 and a scroll bar 25 that scrolls and displays the captured images 24a and 24b. Such a capsule-image display screen allows a real-time visual confirmation about in which posture the in-vivo images are currently taken. In the capsule-image display screen in FIG. 6, the in-vivo images that are taken by the two imaging device of the capsule endoscope 4 are displayed in the same size. Alternatively, an in-vivo image that is taken by a specified imaging device may be displayed in a size larger than an image taken by the other imaging device or an in-vivo image taken by the not-specified imaging device may not be displayed.

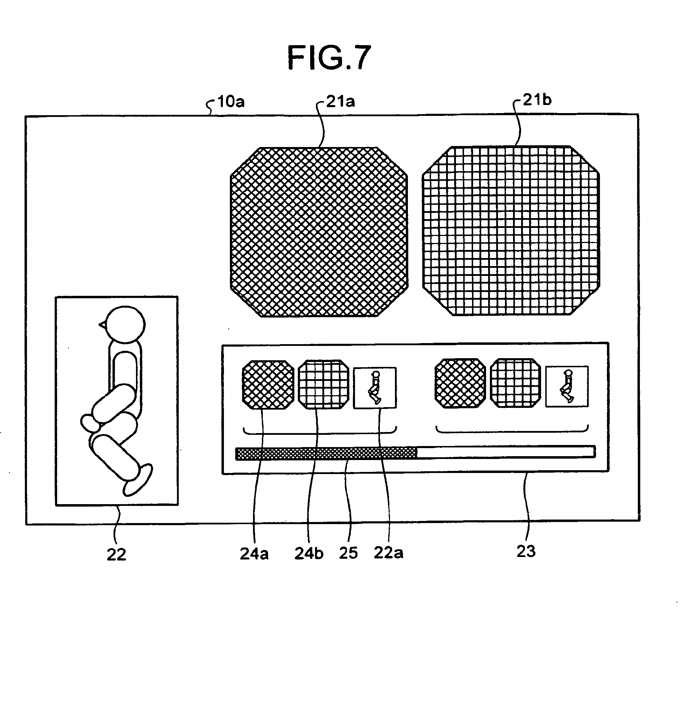

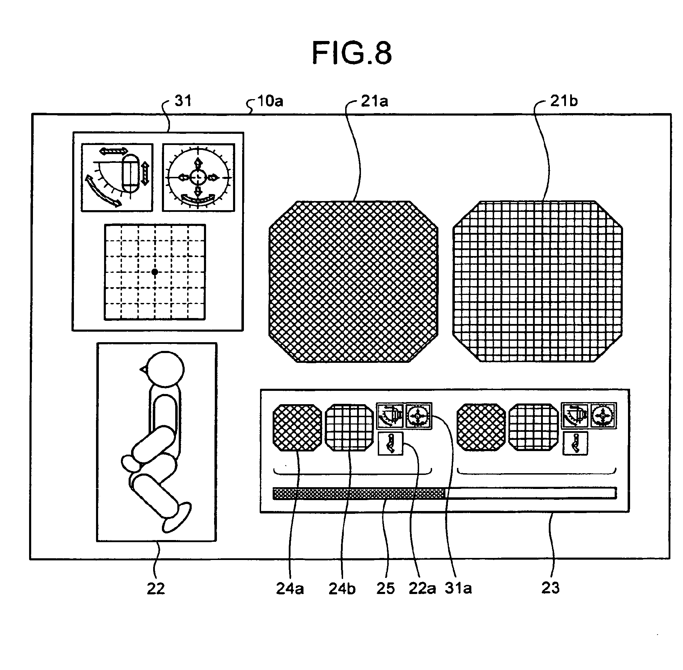

On the capsule-image display screen in FIG. 6, only the posture item of the subject 3 corresponding to the in-vivo images taken by the capsule endoscope 4 is displayed. Alternatively, as illustrated in FIG. 7, the posture item of the subject 3 in which the captured images are taken may be displayed in a posture information display area 22a. Alternatively, as illustrate in FIG. 8, the information on the movement of the capsule endoscope 4 may be displayed on operation information display areas 31 and 31a. In the example of FIG. 8, both of the posture item of the subject 3 and the information on guidance of the capsule endoscope 4 are displayed. Alternatively, only the operation information on the capsule endoscope 4 may be displayed. Information on an estimated position and an estimated posture of the capsule endoscope 4, an operation amount of the input device 9, an imaging device that is specified in the image taking operation, or an operation mode of the capsule endoscope 4 (for example, a mode of operating in liquid or a high-speed operation mode) can be taken as examples of the operation information on the capsule endoscope 4.

### Configuration of diagnostic display screen

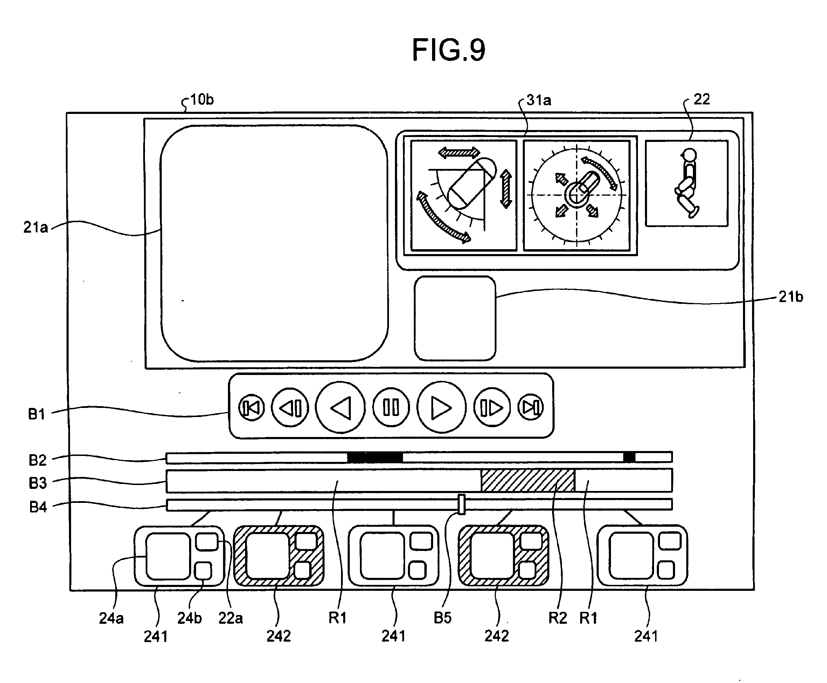

Lastly, with reference to FIG. 9, a configuration will be described of a diagnostic display screen that is displayed on the diagnostic display device 10b when an in-vivo diagnosis is performed on the subject on the basis of the in-vivo images obtained by the above-described diagnostic process.

FIG. 9 is a schematic diagram of a configuration of the diagnostic display screen that is displayed on the diagnostic display device 10b. As illustrated in FIG. 9, the diagnostic display screen includes the capsule image display areas 21a and 21b, the posture information display area 22, the captured-image display area 23, the operation information display area 31a, a play button B1, a red bar B2, an average color bar B3, and a time bar B4. The capsule image display areas 21a and 21b, the posture information display area 22, the captured-image display area 23, and the operation information display area 31 have the same configurations as those of the capsule-image display screen, which is described above, and thus descriptions thereof will be omitted.

The play button B1 is a manipulator for sequentially displaying in-vivo images that are taken in the diagnostic process. The play button B1 is provided with an orderly play button, a reverse play button, and a stop button. The red bar B2 indicates a ratio of red in an in-vivo image that is taken by the capsule endoscope 4. On the basis of the ratio of red, it can be determined whether a bleeding occurs in an observed region. The average color bar B3 indicates the average color of the in-vivo image taken by the capsule endoscope 4. On the basis of the display color of the average color bar B3, it can be determined whether the observed region or the imaging device is changed. Specifically, in the example of FIG. 9, the display color differs between the region R1 and the region R2 of the average color bar B3. Thus, it is known that the imaging device used for taking the image of the region R1 is different from the imaging device used for taking the image of the region R2, e.g., the imaging device used for taking the image of the region R2 is not the specified imaging device. The time bar B4 contains the length of time required for the diagnostic process. The time at which the displayed in-vivo image is taken can be confirmed on the basis of the position of the button B5 in the time bar B4. In the present embodiment, the captured images 24a and 24b and the posture item 22a are displayed in association with the time, on the time bar B4, at which the captured images are captured. In addition, captured images 241 that are specified in a diagnosis and captured images 242 that are specified in a guidance operation are displayed separately. By using such a diagnostic display screen, a doctor can easily confirm in which posture an in-vivo image is captured.

Although the embodiments to which the invention(s) made by the inventor(s) are applied are described above, the present invention is not limited to the description and the drawings constituting a part of the disclosure of the present invention shown by the embodiments. In other words, other embodiments, examples, and operational techniques realized by one ordinarily skilled in the art based on the embodiments described above are all in the scope of the present invention.

### Industrial Applicability

The present invention can be applied to a capsule medical apparatus system that inserts a capsule medical apparatus into a subject to capture an in-vivo image of the subject.

**Reference Signs List**

| | |
|---|---|
| 2 | BED |
| 3 | SUBJECT |
| 4 | CAPSULE ENDOSCOPE |
| 5 | MAGNETIC FIELD GENERATION DEVICE |
| 6 | ANTENNA |
| 7 | RECEIVING DEVICE |
| 8 | CONTROL DEVICE |
| 8a | CONTROL UNIT |
| 8b | STORAGE UNIT |
| 9 | INPUT DEVICE |
| 10a | CAPSULE IMAGE DISPLAY DEVICE |
| 10b | DIAGNOSTIC DISPLAY DEVICE |
| 11 | SIGNAL GENERATOR |

## Claims

1. A medical apparatus system comprising:
a capsule medical apparatus (4) to be inserted into a subject (3), the capsule medical apparatus acquiring information on the subject, wherein acquiring information includes taking an in-vivo image of the subject;
a posture item display unit (10a) that displays multiple recommended posture items of the subject in which the medical apparatus acquires the information on the subject;
an input unit (9) that inputs information on a posture item of the subject in which the information on the subject is acquired among the recommended posture items that are displayed by the posture item display unit;
a display controller (8) that includes recommended-posture information that defines the recommended posture items in which acquisition of the information is recommended and that, in response to the input of the input unit, changes a display mode of the recommended posture item of the subject, which is displayed by the posture item display unit, on the basis of the recommended-posture information, such that the display mode indicates that the posture item is input by the input unit; and
a storage unit that stores the in-vivo image, which is taken by the capsule medical apparatus, in association with the information on the posture of the subject, which is input by the input unit.

2. The medical apparatus system according to claim 1, wherein, when the input unit inputs a posture item of the subject in which acquisition of the information is started, the display controller changes the display mode of the posture item.

3. The medical apparatus system according to claim 2, wherein, when the input unit inputs information indicating completion of the acquisition of the information in the posture item, the display controller further changes the changed display mode of the posture item.

4. The medical apparatus system according to claim 1, wherein the display controller changes the recommended-posture information in accordance with a region of the subject from which the information is acquired.

5. The medical apparatus system according to claim 1, wherein
the recommended-posture information contains information on an order of the posture items, and
the display controller changes the display mode of the posture item, which is displayed by the posture item display unit, on the basis of the information on the order.

6. The medical apparatus system according to claim 5, wherein the display controller changes the display mode of a posture item of the subject in which information is acquired next among the posture items, which are displayed by the posture item display unit, on the basis of the recommended-posture information.

7. The medical apparatus system according to claim 1, further comprising:
an image display unit (10b) that displays the in-vivo image, which is stored in the storage unit, and the information on the posture of the subject, which is associated with the in-vivo image.

8. The medical apparatus system according to claim 7, wherein
the image display unit has a capture function, and
the image display unit includes a captured-image display area (23) in which an in-vivo image (24a, 24b) that is taken by using the capture function and posture information (22a), which is associated with the in-vivo image, are displayed.

9. The medical apparatus system according to claim 7, comprising an operation state confirmation unit that confirms information on operation state of the capsule medical apparatus, wherein
the storage unit stores the information on the operation state of the capsule medical apparatus, which is confirmed by the operation state confirmation unit, in association with the in-vivo image, and
the image display unit displays the in-vivo image (24a, 24b), which is stored in the storage unit, and the information (31a) on the operation state of the capsule medical apparatus, which is associated with the in-vivo image.

10. The medical apparatus system according to claim 8, comprising an operation state confirmation unit that confirms information on operation state of the capsule medical apparatus,
wherein the image display unit displays the information on the operation state of the capsule medical apparatus, which is confirmed by the operation state confirmation unit, in the captured-image display area.

11. The medical apparatus system according to claim 9, wherein
the capsule medical apparatus includes at least one magnetic material, and the capsule medical apparatus system further comprises a guidance magnetic field generation device that acts on the at least one magnetic material to guide the capsule medical apparatus,
wherein the operation state confirmation unit confirms, as the information on the operation state of the capsule medical apparatus, information on a magnetic field generated by the guidance magnetic field generation device.

12. A method of displaying a posture item of a subject, comprising:
acquiring information on a subject with a capsule medical apparatus to be inserted into the subject, where acquiring includes taking an in-vivo image of the subject;
displaying on a posture item display unit multiple recommended posture items of the subject in which the medical apparatus acquires the information on the subject;
inputting information on a posture item of the subject in which the information on the subject is acquired among the recommended posture items that are displayed;
changing, in response to the input and on the basis of the recommended posture information that defines the recommended posture items in which acquisition of the information is recommended, a display mode of the posture item of the subject that is displayed, such that the display mode indicates that the posture item is input; and
storing the taken in-vivo image in association with the information on the posture of the subject which is input.

## Patentansprüche

1. Medizinisches Vorrichtungssystem, aufweisend:
eine medizinische Kapselvorrichtung (4), die in einen Probanden (3) eingeführt wird, wobei die medizinische Kapselvorrichtung Information über den Probanden erlangt, wobei das Erlangen von Information ein Aufnehmen eines In-vivo-Bilds des Probanden einschließt;
eine Haltungsanzeigeeinheit (10a), die mehrere vorgeschlagene Haltungen des Probanden, in denen die medizinische Vorrichtung die Information über den Probanden erlangt, anzeigt;
eine Eingabeeinheit (9), die Information über eine Haltung des Probanden, in der die Information über den Probanden erlangt wird, aus den vorgeschlagenen Haltungen, die durch die Haltungsanzeigeeinheit angezeigt werden, eingibt;
eine Anzeigesteuerung (8), die Informationen zu vorgeschlagenen Haltungen, die die vorgeschlagenen Haltungen, in denen ein Erlangen der Information vorgeschlagen wird, definieren, einschließt, und die in Reaktion auf die Eingabe der Eingabeeinheit einen Anzeigemodus der vorgeschlagenen Haltung des Probanden, die durch die Haltungsanzeigeeinheit angezeigt wird, auf der Basis der Informationen zu vorgeschlagenen Haltungen ändert, so dass der Anzeigemodus angibt, dass die Haltung durch die Eingabeeinheit eingegeben ist; und
eine Speichereinheit, die das In-vivo-Bild, das durch die medizinische Kapselvorrichtung aufgenommen wurde, in Verbindung mit der Information über die Haltung des Probanden, die durch die Eingabeeinheit eingegeben wurde, speichert.

2. Medizinisches Vorrichtungssystem gemäß Anspruch 1, wobei, wenn die Eingabeeinheit eine Haltung des Probanden, in der das Erlangen der Information gestartet wird, eingibt, die Anzeigesteuerung den Anzeigemodus der Haltung ändert.

3. Medizinisches Vorrichtungssystem gemäß Anspruch 2, wobei, wenn die Eingabeeinheit Information, die einen Abschluss des Erlangens von Information in der Haltung angibt, eingibt, die Anzeigesteuerung den geänderten Anzeigemodus der Haltung weiter ändert.

4. Medizinisches Vorrichtungssystem gemäß Anspruch 1, wobei die Anzeigesteuerung die Informationen zu vorgeschlagenen Haltungen im Einklang mit einer Region des Probanden, von der die Information erlangt wird, ändert.

5. Medizinisches Vorrichtungssystem gemäß Anspruch 1, wobei
die Informationen zu vorgeschlagenen Haltungen Informationen zu einer Reihenfolge der Haltungen enthalten, und
die Anzeigesteuerung den Anzeigemodus der Haltung, die durch die Haltungsanzeigeeinheit angezeigt wird, auf Basis der Informationen zu der Reihenfolge ändert.

6. Medizinisches Vorrichtungssystem gemäß Anspruch 5, wobei die Anzeigesteuerung den Anzeigemodus einer Haltung des Probanden, in der als nächstes Information erlangt wird, aus den Haltungen, die durch die Haltungsanzeigeeinheit angezeigt werden, auf Basis der Informationen zu vorgeschlagenen Haltungen ändert.

7. Medizinisches Vorrichtungssystem gemäß Anspruch 1, des Weiteren aufweisend:
eine Bildanzeigeeinheit (10b), die das In-vivo-Bild, das in der Speichereinheit gespeichert ist, und die Information über die Haltung des Probanden, die in Verbindung mit dem In-vivo-Bild steht, anzeigt.

8. Medizinisches Vorrichtungssystem gemäß Anspruch 7, wobei
die Bildanzeigeeinheit eine Festhaltefunktion hat, und
die Bildanzeigeeinheit einen Anzeigebereich (23) für ein festgehaltenes Bild einschließt, in dem ein In-vivo-Bild (24a, 24b), das durch Verwendung der Festhaltefunktion aufgenommen wurde, und Haltungsinformation (22a), die in Verbindung mit dem In-vivo-Bild steht, angezeigt werden.

9. Medizinisches Vorrichtungssystem gemäß Anspruch 7, das eine Betriebsstatusbestätigungseinheit aufweist, die Information über einen Betriebsstatus der medizinischen Kapselvorrichtung bestätigt, wobei
die Speichereinheit die Information über den Betriebsstatus der medizinischen Kapselvorrichtung, der durch die Betriebsstatusbestätigungseinheit bestätigt wird, in Verbindung mit dem In-vivo-Bild speichert, und
die Bildanzeigeeinheit das In-vivo-Bild (24a, 24b), das in der Speichereinheit gespeichert ist, und die Information (31a) über den Betriebsstatus der medizinischen Kapselvorrichtung, der in Verbindung mit dem In-vivo-Bild steht, anzeigt.

10. Medizinisches Vorrichtungssystem gemäß Anspruch 8, das eine Betriebsstatusbestätigungseinheit aufweist, die Information über einen Betriebsstatus der medizinischen Kapselvorrichtung bestätigt,
wobei die Bildanzeigeeinheit die Information über den Betriebsstatus der medizinischen Kapselvorrichtung, der durch die Betriebsstatusbestätigungseinheit bestätigt wird, in dem Anzeigebereich für ein festgehaltenes Bild anzeigt

11. Medizinisches Vorrichtungssystem gemäß Anspruch 9, wobei
die medizinische Kapselvorrichtung mindestens ein magnetisches Material einschließt, und
das medizinische Kapselvorrichtungssystem des Weiteren ein Leitmagnetfelderzeugungsgerät, das auf das mindestens eine magnetische Material einwirkt, um die medizinische Kapselvorrichtung zu leiten, aufweist,
wobei die Betriebsstatusbestätigungseinheit Information über ein Magnetfeld, das durch das Leitmagnetfelderzeugungsgerät erzeugt wird, als die Information über den Betriebsstatus der medizinischen Kapselvorrichtung bestätigt.

12. Verfahren zum Anzeigen einer Haltung eines Probanden, aufweisend:
Erlangen von Information über einen Probanden mit einer medizinischen Kapselvorrichtung, die in den Probanden eingeführt wird, wobei das Erlangen ein Aufnehmen eines In-vivo-Bilds des Probanden einschließt;
Anzeigen auf einer Haltungsanzeigeeinheit mehrere vorgeschlagene Haltungen des Probanden, in denen die medizinische Vorrichtung die Information über den Probanden erlangt;
Eingeben von Information über eine Haltung des Probanden, in der die Information über den Probanden erlangt wird, aus den vorgeschlagenen Haltungen, die angezeigt werden;
Ändern, in Reaktion auf die Eingabe und auf der Basis der Informationen zu vorgeschlagenen Haltungen, die die vorgeschlagenen Haltungen, in denen ein Erlangen der Information vorgeschlagen wird, definieren, eines Anzeigemodus der Haltung des Probanden, die angezeigt wird, so dass der Anzeigemodus angibt, dass die Haltung eingegeben ist; und
Speichern des aufgenommenen In-vivo-Bilds in Verbindung mit der Information über die Haltung des Probanden, die eingegeben wurde.

## Revendications

1. Système d'appareil médical comprenant :
un appareil médical de type capsule (4) destiné à être inséré à l'intérieur d'un sujet (3), l'appareil médical de type capsule acquérant des informations sur le sujet, dans lequel l'acquisition d'informations inclut la prise d'une image in vivo du sujet ;
une unité d'affichage (10a) d'éléments de posture qui affiche des éléments de posture recommandés multiples du sujet dans lequel l'appareil médical acquiert les informations sur le sujet ;
une unité d'entrée (9) qui entre des informations sur un élément de posture du sujet, dans lequel les informations sur le sujet sont acquises parmi les éléments de posture recommandés qui sont affichés par l'unité d'affichage d'éléments de posture ;
un contrôleur (8) d'affichage qui inclut des informations de posture recommandée qui définissent les éléments de posture recommandés dans lesquels une acquisition des informations est recommandée et qui, en réponse à l'entrée de l'unité d'entrée, change un mode d'affichage de l'élément de posture recommandé du sujet, qui est affiché par l'unité d'affichage d'éléments de posture, sur la base des informations de posture recommandée, de telle sorte que le mode d'affichage indique que l'élément de posture est entré par l'unité d'entrée ; et
une unité de stockage qui stocke l'image in vivo, qui est prise par l'appareil médical de type capsule, en association avec les informations sur la posture du sujet, qui sont entrées par l'unité d'entrée.

2. Système d'appareil médical selon la revendication 1, dans lequel, lorsque l'unité d'entrée entre un élément de posture du sujet dans lequel une acquisition des informations est commencée, le contrôleur d'affichage change le mode d'affichage de l'élément de posture.

3. Système d'appareil médical selon la revendication 2, dans lequel, lorsque l'unité d'entrée entre des informations indiquant la fin de l'acquisition des informations sur l'élément de posture, le contrôleur d'affichage change à nouveau le mode d'affichage changé de l'élément de posture.

4. Système d'appareil médical selon la revendication 1, dans lequel le contrôleur d'affichage change les informations de posture recommandée en fonction d'une région du sujet de laquelle les informations sont acquises.

5. Système d'appareil médical selon la revendication 1, dans lequel
les informations de posture recommandée contiennent des informations sur un ordre des éléments de posture, et
le contrôleur d'affichage change le mode d'affichage de l'élément de posture, qui est affiché par l'unité d'affichage d'éléments de posture, sur la base des informations relatives à l'ordre.

6. Système d'appareil médical selon la revendication 5, dans lequel le contrôleur d'affichage change le mode d'affichage d'un élément de posture du sujet dans lequel des informations sont acquises ensuite parmi les éléments de posture, qui sont affichés par l'unité d'affichage d'éléments de posture, sur la base des informations de posture recommandée.

7. Système d'appareil médical selon la revendication 1, comprenant en outre :
une unité d'affichage (10b) d'image qui affiche l'image in vivo, qui est stockée dans l'unité de stockage, et les informations sur la posture du sujet, qui sont associées avec l'image in vivo.

8. Système d'appareil médical selon la revendication 7, dans lequel
l'unité d'affichage d'image a une fonction de capture, et
l'unité d'affichage d'image inclut une zone d'affichage (23) d'image capturée dans laquelle une image in vivo (24a, 24b) qui est prise en utilisant la fonction de capture et les informations de posture (22a), qui sont associées avec l'image in vivo, sont affichées.

9. Système d'appareil médical selon la revendication 7, comprenant une unité de confirmation d'état opérationnel qui confirme des informations sur un état opérationnel de l'appareil médical de type capsule, dans lequel
l'unité de stockage stocke les informations sur l'état opérationnel de l'appareil médical de type capsule, qui est confirmé par l'unité de confirmation d'état opérationnel, en association avec l'image in vivo, et
l'unité d'affichage d'image affiche l'image in vivo (24a, 24b), qui est stockée dans l'unité de stockage, et les informations (31a) sur l'état opérationnel de l'appareil médical de type capsule, qui sont associées avec l'image in vivo.

10. Système d'appareil médical selon la revendication 8, comprenant une unité de confirmation d'état opérationnel qui confirme des informations sur un état opérationnel de l'appareil médical de type capsule,
dans lequel l'unité d'affichage d'image affiche les informations sur l'état opérationnel de l'appareil médical de type capsule, qui est confirmé par l'unité de confirmation d'état opérationnel, dans la zone d'affichage d'image capturée.

11. Système d'appareil médical selon la revendication 9, dans lequel
l'appareil médical de type capsule inclut au moins un matériau magnétique ; et
le système d'appareil médical de type capsule comprend en outre un dispositif de génération de champ magnétique de guidage qui agit sur l'au moins un matériau magnétique pour guider l'appareil médical de type capsule,
dans lequel l'unité de confirmation d'état opérationnel confirme, comme les informations sur l'état opérationnel de l'appareil médical de type capsule, des informations sur un champ magnétique généré par le dispositif de génération de champ magnétique de guidage.

12. Procédé d'affichage d'un élément de posture d'un sujet, comprenant :
l'acquisition d'informations sur un sujet avec un appareil médical de type capsule destiné à être inséré à l'intérieur du sujet, où l'acquisition inclut la prise d'une image in vivo du sujet ;
l'affichage sur une unité d'affichage d'éléments de posture d'éléments de posture recommandés multiples du sujet dans lequel l'appareil médical acquiert les informations sur le sujet ;
l'entrée d'informations sur un élément de posture du sujet dans lequel les informations sur le sujet sont acquises parmi les éléments de posture recommandés qui sont affichés ;
le changement, en réponse à l'entrée et sur la base des informations de posture recommandée qui définissent les éléments de posture recommandés dans lesquels une acquisition des informations est recommandée, d'un mode d'affichage de l'élément de posture du sujet qui est affiché, de telle sorte que le mode d'affichage indique que l'élément de posture est entré ; et
le stockage de l'image in vivo prise en association avec les informations sur la posture du sujet qui sont entrées.
